# FASCICULE DE BREVET EUROPEEN

(11) **EP 1 165 483 B1**
(45) Date de publication et mention de la délivrance du brevet: **03.03.2004**
(21) Numéro de dépôt: 00910982.8
(22) Date de dépôt: 17.03.2000
(51) Int. Cl.: C07C 68/02, C07C 69/96, C01B 31/00

(54) **PROCEDE DE PREPARATION DU FLUORURE DE CARBONYLE**
VERFAHREN ZUR HERSTELLUNG VON CARBONYLFLUORID
METHOD FOR THE PRODUCTION OF CARBONYL FLUORIDE

(30) Priorité: 02.04.1999 FR 9904125
(43) Date de publication de la demande: 02.01.2002
(60) Demande divisionnaire: 03026475.8
(73) Titulaire: ISOCHEM, 75194 Paris Cedex 04 (FR)
(72) Inventeur: DELABROUILLE, Philippe, F-91150 Brouy (FR); GRENOUILLAT, Denis, F-91200 Athis-Mons (FR); SENET, Jean-Pierre, F-77760 La Chapelle la Reine (FR); SENNYEY, Gérard, F-91190 Gif-sur-Yvette (FR)
(74) Mandataire: Pech, Bernard
(86) Numéro de dépôt international: PCT/FR2000/000662
(87) Numéro de publication internationale: WO 2000/059859

(56) Documents cités:
- JOHN CUOMO ET AL.: "An Efficient and Convenient Synthesis of Fluoroformates and Carbamoyl Fluorides" JOURNAL OF ORGANIC CHEMISTRY., vol. 44, no. 6, 16 mars 1979 (1979-03-16), pages 1016-1017, XP002134146 AMERICAN CHEMICAL SOCIETY. EASTON., US ISSN: 0022-3263
- P.E.ALDRICH ET AL.: "Alpha-fluorinated Ethers. II. Alkyl Fluoroalkyl Ethers" JOURNAL OF ORGANIC CHEMISTRY., vol. 29, no. 1, 13 janvier 1964 (1964-01-13), pages 11-15, XP002140047 AMERICAN CHEMICAL SOCIETY. EASTON., US ISSN: 0022-3263
- F.S.FAWCETT ET AL.: "The Chemistry of Carbonyl Fluoride. I. The Fluorination of Organic Compounds" JOURNAL OF THE AMERICAN CHEMICAL SOCIETY., vol. 84, no. 22, 20 novembre 1962 (1962-11-20), pages 4275-4285, XP002140048 AMERICAN CHEMICAL SOCIETY, WASHINGTON, DC., US ISSN: 0002-7863

## Description

La présente invention concerne la préparation de fluorure de carbonyle de très grande pureté, particulièrement utile pour réagir avec les alcools aliphatiques afin d'obtenir des fluoroformiates aliphatiques.

Dans l'article "the Chemistry of Carbonyl Fluoride" par F.S. FAWCETT et al,Journal of the American Chemical Society, vol. 84, n° 22, 20 novembre 1962, pages 4275-4285, il est décrit un procédé de préparation du fluorure de carbonyle par réaction du phosgène dans l'acétonitrile avec du fluorure de sodium. Le fluorure de carbonyle est cependant obtenu avec une faible conversion, de 70 à 80 % et une pureté de 95 % seulement.

Il existait donc un besoin d'un procédé de préparation du fluorure de carbonyle avec un excellent rendement et une pureté améliorée.

Selon le procédé de l'invention, le fluorure de carbonyle est obtenu en faisant réagir du phosgène, du diphosgène ou du triphosgène, ou un de leurs mélanges, avec un excès de fluorure de sodium en poudre dont les grains ont une surface spécifique supérieure ou égale à 0,1 m²/g et/ou un diamètre moyen inférieur ou égal à 20 µm, dans un solvant choisi parmi les solvants polaires et aprotiques, à une température comprise entre environ 25°C et environ 120°C, puis en faisant passer les gaz présents dans un condenseur dont la température est comprise entre environ 0° et environ - 50°C.

En réalisant le procédé de préparation du fluorure de carbonyle avec l'ensemble de ces conditions, on obtient à la sortie du condenseur, du fluorure de carbonyle avec une pureté très élevée, ne contenant pas de fluorochlorure de carbonyle et pratiquement pas de phosgène.

L'absence de ces deux gaz est particulièrement intéressante car on évite ainsi la formation de chloroformiates comme sous-produits, ce qui entraînait auparavant une diminution des rendements obtenus en fluoroformiates. De plus, les chloroformiates sont des composés très instables et on évite ainsi les risques de décomposition violente.

Les caractéristiques de la poudre de fluorure de sodium sont importantes pour une bonne réalisation de ce procédé. On a en effet constaté que lorsque les grains de fluorure de sodium ne présentent pas les caractéristiques décrites précédemment, la pureté du fluorure de carbonyle est nettement moins élevée et les rendements en fluorure de carbonyle et en fluoroformiates sont nettement plus faibles.

De préférence, les grains de fluorure de sodium ont une surface spécifique supérieure à 0,1 m²/g et de façon encore plus préférée également un diamètre moyen inférieur à 20 µm.

La poudre de fluorure de sodium doit être en excès par rapport au phosgène. De préférence, on utilise une quantité de 3 à 5 moles de fluorure de sodium par mole de phosgène.

Le solvant, bien sûr inerte vis à vis des réactifs, est choisi parmi les solvants qui sont aprotiques et polaires, c'est à dire les solvants dont la constante diélectrique est supérieure à 10 et de préférence supérieure à 20. Les nitriles aliphatiques conviennent bien. De préférence, on utilise l'acétonitrile.

La température du milieu réactionnel est de préférence comprise entre environ 35° et 80°C. La température du condenseur est quant à elle en particulier comprise entre environ - 20°C et - 40°C.

Le phosgène et/ou ses précurseurs sont de préférence introduits progressivement, dans le milieu réactionnel. Le phosgène est en général utilisé sous forme gazeuse. Il peut aussi être introduit sous forme de solution dans le solvant.

Le diphosgène ou le triphosgène sont introduits, en général en phase liquide, éventuellement en solution dans le solvant, en quantités suffisantes pour donner la quantité de phosgène souhaitée.

La réaction est de préférence réalisée avec des composés et dans des conditions anhydres.

Le fluorure de carbonyle obtenu à la sortie du condenseur ne contient pas de fluorochlorure de carbonyle. Il contient d'infimes quantités de phosgène. Sa pureté déterminée par chromatographie en phase gazeuse est le plus souvent supérieure à 99% et son rendement est généralement supérieur à 95%.

Ce fluorure de carbonyle peut être directement utilisé pour préparer les fluoroformiates et de préférence il est mis à réagir au fur et à mesure de sa formation. La réaction du phosgène avec le fluorure de sodium est alors effectuée dans un premier réacteur, à une température de préférence comprise entre environ 35°C et 80°C. On utilise une quantité de phosgène au moins stoechiométrique par rapport à l'alcool que l'on souhaite transformer, et de préférence de 1,1 à 2 moles de phosgène par mole de l'alcool.

La quantité de fluorure de sodium que l'on fait réagir avec le phosgène est dans ce cas de préférence de 3 à 6 moles par mole de l'alcool à transformer et la quantité de solvant pour cette première réaction est généralement de 0,3 à 0,6 litre par mole de l'alcool.

Les gaz qui se dégagent du milieu réactionnel passent à travers le condenseur et sont introduits au fur et à mesure dans la solution de l'alcool contenue dans le deuxième réacteur.

La température du condenseur est de préférence comprise entre environ - 20°C et - 40°C. Les liquides condensés par le condenseur sont généralement recyclés dans le premier réacteur.

Le fluorure de sodium utilisé dans le deuxième réacteur est de préférence un fluorure de sodium ayant les mêmes caractéristiques que celui utilisé dans le premier réacteur.

Cette méthode préférée de préparation des fluoroformiates présente de grands avantages. Les manipulations sont réduites. Le procédé est plus économique et plus simple. Les rendements sont excellents et voisins de 100%.

Le procédé à partir du phosgène dure, en général, quelques heures. Lorsque la réaction est terminée, on sépare la solution de fluoroformiate du milieu réactionnel, généralement par filtration.

Le procédé est illustré par les exemples qui suivent.

Sauf mention contraire, dans ces exemples, les réactions de préparation du fluorure de carbonyle et son utilisation pour la préparation des fluoroformiates aliphatiques sont effectuées avec des composés, des appareillages et dans des conditions anhydres.

### EXEMPLE 1 :

Dans un premier réacteur, on a placé 189 g (4,5 moles) de fluorure de sodium en poudre dont les grains ont un diamètre moyen de 8,6 µm et une surface spécifique de 0,27 m²/g et 340 ml d'acétonitrile. Ce premier réacteur est surmonté d'un condenseur maintenu à - 30°C qui est relié à un deuxième réacteur dans lequel on a placé 74 g (1 mole) de tertiobutanol et 49 g (1,17 mole) de fluorure de sodium de mêmes caractéristiques que précédemment et 150 ml de tétraglyme (diméthyléther du tétraéthylèneglycol), les deux réacteurs sont munis d'un système d'agitation. On chauffe le premier réacteur à une température de 50° C et la température du deuxième réacteur est maintenue aux environs de + 5°C. On introduit progressivement dans le milieu solvant, 148,5 g (1,5 mole) de phosgène gazeux pendant environ 4 heures. On analyse par chromatographie gazeuse et spectroscopie de masse, les gaz à la sortie du condenseur. On ne trouve pas trace de fluorochlorure de carbonyle et seulement des traces de phosgène en quantité inférieure à 0,1% en masse. La pureté du fluorure de carbonyle est supérieure à 99%. Le rendement déterminé par analyse des sels restants est de 98%.

L'obtention du fluoroformiate de tertiobutyle terminée, les gaz sont éliminés par un courant d'azote. Le contenu du deuxième réacteur est filtré et le gâteau est rincé avec quelques millilitres de tétraglyme.

Par analyse RMN ¹H, on constate que la conversion en fluoroformiate de tertiobutyle est de 100 %.

### EXEMPLE 2 : Exemple comparatif

Pour cet exemple, on utilise le fluorure de carbonyle le plus pur vendu dans des bouteilles acier et sous pression par la Société Union Carbide.

On relie cette bouteille à un réacteur de même type que le deuxième réacteur de l'exemple précédent qui contient les mêmes quantités de composés avec les mêmes caractéristiques et on opère dans les mêmes conditions. On introduit progressivement 1 mole de fluorure de carbonyle.

On constate que la conversion (déterminée par analyse RMN ¹H) en fluoroformiate de tertiobutyle est alors de 93%.

### EXEMPLE 3 :

Dans un premier réacteur, on a placé 30 g (0,7 mole) de fluorure de sodium dont les grains ont un diamètre moyen de 15 µm et une surface spécifique de 0,2 m²/g et 76 ml d'acétonitrile, et dans un deuxième réacteur, on a placé 11,1 g (0,15 mole) de tertiobutanol, 11 g (0,26 mole) de fluorure de sodium de mêmes caractéristiques que celui du premier réacteur et 25 ml de monoglyme (diméthoxyéthane). Les deux réacteurs sont reliés comme précédemment par l'intermédiaire d'un condenseur à - 30°C. On chauffe le premier réacteur à une température de 55° à 60°C et on maintient le deuxième réacteur à une température de 20° à 25°C. On introduit dans le milieu réactionnel, 18,5 g (0,19 mole) de phosgène gazeux en trois heures. La réaction terminée, on fait passer un courant d'azote. On filtre le mélange réactionnel issu du deuxième réacteur sur une précouche de fluorure de sodium de mêmes caractéristiques. On rince le gâteau avec quelques millilitres de monoglyme. On recueille ainsi le fluoroformiate de tertiobutyle en solution dans le monoglyme. La quantité obtenue de ce fluoroformiate déterminée par analyse chromatographique en phase gazeuse est de 18 g soit un rendement de 100 %. On ajoute à cette solution, 0,36 g de diméthylformamide. La solution a pu être conservée 6 mois à une température comprise entre 0° et 5°C.

### EXEMPLE 4 :

Dans le premier réacteur, on a placé 75,6 g (1,8 mole) de fluorure de sodium dont les grains ont un diamètre moyen de 12 µm et une surface spécifique de 0,23 m²/g et 100 ml d'acétonitrile. Dans le deuxième réacteur, on a placé 22,2 g (0,3 mole) de tertiobutanol, 14,7 g (0,35 mole) de fluorure de sodium identique à celui du premier réacteur et 40 ml de tétraglyme. On chauffe le premier réacteur à 80°C, on maintient à - 30°C la température du condenseur et à 5°C la température du deuxième réacteur. On introduit en moins d'une heure dans le premier réacteur, 44,6 g (0,15 mole) de triphosgène dans 100 ml d'acétonitrile. On laisse réagir pendant deux heures et on dose le fluoroformiate formé par RMN ¹H. La conversion en fluoroformiate de tertiobutyle est de 100 %.

Dans un autre essai, on a remplacé le triphosgène par une quantité équivalente de diphosgène. Les résultats obtenus sont identiques.

### EXEMPLE 5 :

On opère comme à l'exemple 1, avec dans le premier réacteur 168 g (4 moles) de fluorure de sodium en poudre possédant les mêmes caractéristiques que décrites à l'exemple 1 et 320 ml d'acétonitrile et dans le deuxième réacteur, 108 g (1 mole) d'alcool benzylique, 50,5 g (1,2 mole) de fluorure de sodium de mêmes caractéristiques que précédemment et 150 g de diméthoxyéthane.

Après introduction de 120 g de phosgène, dégazage et filtration de la suspension contenue dans le second réacteur, on élimine le solvant par évaporation sous pression réduite puis on effectue une distillation fractionnée. On recueille ainsi 137 g de fluoroformiate de benzyle( rendement 89%), liquide incolore, dont les caractéristiques sont les suivantes :
Point d'ébullition : 64°C / 4 mm Hg,
RMN ¹H (CCl₄) δ : 7,42(s, 5H), 5,25 (s, 2H)

### EXEMPLE 6 :

On opère comme à l'exemple précédent, le fluorure de sodium utilisé étant identique, mais avec, dans le premier réacteur 84 g ( 2 moles) de fluorure de sodium et 170 g d'acétonitrile et dans le deuxième réacteur 76 g (0,5 mole) de 1-adamantanol, 25 g (0,6 mole) de fluorure de sodium ainsi que 100 g de diméthoxyéthane.

Après introduction de 62 g de phosgène, dégazage et filtration de la suspension contenue dans le deuxième réacteur, on élimine le solvant par évaporation à 45°C sous 0,1 mm Hg. On recueille ainsi 90 g ( rendement 91%) de fluoroformiate de 1-adamantyle, produit solide, avec les caractéristiques suivantes :
Point de fusion : 32°-33°C,
Spectre IR : 1830 cm⁻¹.

### EXEMPLE 7 :

On opère comme à l'exemple 1 mais avec du fluorure de sodium dont les grains ont un diamètre moyen de 9,5 µm et une surface spécifique de 0,25 m²/g.

Le premier réacteur contient 160 g ( 3,8 moles) de fluorure de sodium et 310 ml d'acétonitrile et le second réacteur 196 g ( 1 mole ) de 9-fluorénylméthanol à 99,5% (HPLC), 50 g (1,19 mole ) de fluorure de sodium et 340 g de diméthoxyéthane. Après introduction de 120 g de phosgène dans le premier réacteur, dégazage et filtration du contenu du second réacteur, on recueille environ 570 g d'une solution limpide de couleur marron clair. La conversion en Fmoc-F (déterminée par analyse RMN ¹H) est de 100%.

On ajoute à 200 g de cette solution chauffée à 50°C, 200 ml d'Isopar G également chauffés à 50°C et on concentre le tout jusqu'à 220 ml en maintenant la température toujours supérieure à 30° C. On filtre ensuite sur célite à une température toujours supérieure à 30°C, on rince le gâteau avec 50 ml d'essence G à une température supérieure à 30°C. On refroidit ensuite lentement le filtrat à 0°C, on filtre les cristaux obtenus, on rince deux fois avec de l'essence G à 0°C (100 ml et 50 ml). Après séchage à 20°-30°C, on obtient 58,5 g (rendement global 69%) d'un produit cristallisé blanc de point de fusion 41°C et de titre en Fmoc-F supérieur à 99% (déterminé par analyse HPLC).

## Revendications

1. Procédé de préparation du fluorure de carbonyle, **caractérisé en ce qu'**on fait réagir du phosgène, du diphosgène ou du triphosgène, ou un de leurs mélanges, avec un excès de fluorure de sodium en poudre dont les grains ont une surface spécifique supérieure ou égale à 0,1 m²/g et/ou un diamètre moyen inférieur ou égal à 20 µm, dans un solvant choisi parmi les solvants polaires et aprotiques, à une température comprise entre 25° et 120°C, puis on fait passer les gaz présents dans un condenseur dont la température est comprise entre 0° et - 50°C.

2. Procédé selon la revendication 1, **caractérisé en ce que** les grains de fluorure de sodium ont une surface spécifique supérieure ou égale à 0,1 m²/g.

3. Procédé selon la revendication 1 ou 2, **caractérisé en ce que** les grains de fluorure de sodium ont un diamètre moyen inférieur ou égal à 20 µm.

4. Procédé selon l'une quelconque des revendications précédentes, **caractérisé en ce que** la quantité de fluorure de sodium mis à réagir avec le phosgène est de 3 à 5 moles par mole de phosgène.

5. Procédé selon l'une quelconque des revendications précédentes, **caractérisé en ce que** le phosgène et/ou ses précurseurs sont introduits progressivement.

6. Procédé selon l'une quelconque des revendications précédentes, **caractérisé en ce que** le solvant est l'acétonitrile.

7. Procédé selon l'une quelconque des revendications précédentes, **caractérisé en ce que** l'on opère avec des composés et dans des conditions anhydres.

8. Procédé selon l'une quelconque des revendications précédentes, **caractérisé en ce que** les liquides condensés par le condenseur sont recyclés dans le milieu réactionnel.

9. Procédé selon l'une quelconque des revendications précédentes, **caractérisé en ce qu'**on fait réagir le phosgène avec le fluorure de sodium.

## Claims

1. Process for preparing carbonyl fluoride, **characterized in that** phosgene, diphosgene or triphosgene, or a mixture thereof, is reacted with an excess of sodium fluoride powder, the grains of which have a specific surface area of greater than or equal to 0.1 m²/g and/or a mean diameter of less than or equal to 20 µm, in a solvent chosen from polar, aprotic solvents, at a temperature of between 25 and 120°C, after which the gases present are passed into a condenser whose temperature is between 0 and -50°C.

2. Process according to Claim 1, **characterized in that** the sodium fluoride grains have a specific surface area of greater than or equal to 0.1 m²/g.

3. Process according to Claim 1 or 2, **characterized in that** the sodium fluoride grains have a mean diameter of less than or equal to 20 µm.

4. Process according to any one of the preceding claims, **characterized in that** the amount of sodium fluoride reacted with the phosgene is from 3 to 5 mol per mole of phosgene.

5. Process according to any one of the preceding claims, **characterized in that** the phosgene and/or precursors thereof is (are) introduced gradually.

6. Process according to any one of the preceding claims, **characterized in that** the solvent is acetonitrile.

7. Process according to any one of the preceding claims, **characterized in that** it is performed with anhydrous compounds and under anhydrous conditions.

8. Process according to any one of the preceding claims, **characterized in that** the liquids condensed by the condenser are recycled into the reaction medium.

9. Process according to any one of the preceding claims, **characterized in that** the phosgene is reacted with the sodium fluoride.

## Patentansprüche

1. Verfahren zur Herstellung von Carbonylfluorid, **dadurch gekennzeichnet, dass** Phosgen, Diphosgen oder Triphosgen oder eines der Gemische dieser Verbindungen mit einem Überschuss an pulverförmigem Natriumfluorid, dessen Partikel eine spezifische Oberfläche von 0,1 m²/g oder darüber und/oder einen mittleren Durchmesser von 20 µm oder darunter aufweisen, in einem Lösemittel, das unter den polaren und aprotischen Lösemitteln ausgewählt wird, bei einer Temperatur, die im Bereich von 25 bis 120 °C liegt, umgesetzt wird, wonach die vorhandenen Gase in einen Kühler geleitet werden, dessen Temperatur im Bereich von 0 bis -50 °C liegt.

2. Verfahren nach Anspruch 1, **dadurch gekennzeichnet, dass** die Natriumfluoridpartikel eine spezifische Oberfläche von 0,1 m²/g oder darüber aufweisen.

3. Verfahren nach Anspruch 1 oder 2, **dadurch gekennzeichnet, dass** die Natriumfluoridpartikel einen mittleren Durchmesser von 20 µm oder darunter aufweisen.

4. Verfahren nach einem der vorhergehenden Ansprüche, **dadurch gekennzeichnet, dass** die Menge an Natriumfluorid, die mit dem Phosgen umgesetzt wird, 3 bis 5 mol pro Mol Phosgen beträgt.

5. Verfahren nach einem der vorhergehenden Ansprüche, **dadurch gekennzeichnet, dass** das Phosgen und/oder seine Vorläufer nach und nach zugegeben werden.

6. Verfahren nach einem der vorhergehenden Ansprüche, **dadurch gekennzeichnet, dass** es sich bei dem Lösemittel um Acetonitril handelt.

7. Verfahren nach einem der vorhergehenden Ansprüche, **dadurch gekennzeichnet, dass** es mit wasserfreien Verbindungen unter wasserfreien Bedingungen durchgeführt wird.

8. Verfahren nach einem der vorhergehenden Ansprüche, **dadurch gekennzeichnet, dass** die durch den Kühler kondensierten Flüssigkeiten in das Reaktionsmedium zurückgeführt werden.

9. Verfahren nach einem der vorhergehenden Ansprüche, **dadurch gekennzeichnet, dass** Phosgen mit Natriumfluorid umgesetzt wird.
